# EUROPÄISCHE PATENTSCHRIFT

(11) **EP 1 349 611 B1**
(45) Veröffentlichungstag und Bekanntmachung des Hinweises auf die Patenterteilung: **08.03.2006**
(21) Anmeldenummer: 02701174.1
(22) Anmeldetag: 04.01.2002
(51) Int. Cl.: A61N 5/06

(54) **STERILES GEWEBEZUGANGSSYSTEM**
STERILE TISSUE ACCESS SYSTEM
SYSTEME STERILE D'ACCES AUX TISSUS

(30) Priorität: 08.01.2001 DE 10101143
(43) Veröffentlichungstag der Anmeldung: 08.10.2003
(73) Patentinhaber: Speck, Ulrich, D-13465 Berlin (DE)
(72) Erfinder: SPECK, Ulrich, Prof. Dr., 13465 Berlin (DE); HOSTEN, Norbert, Prof. Dr. med., 10711 Berlin (DE); LUKIC, Goran, 8180 Bülach (CH)
(74) Vertreter: Wablat, Wolfgang
(86) Internationale Anmeldenummer: PCT/DE2002/000038
(87) Internationale Veröffentlichungsnummer: WO 2002/053230

(56) Entgegenhaltungen:
- WO-A-94/26184
- DE-A- 4 322 955
- DE-A- 19 500 353
- DE-A- 19 702 898
- US-A- 5 222 953

## Beschreibung

Die Erfindung betrifft ein steriles Gewebezugangs- und - behandlungssystem gemäß den Merkmalen des Oberbegriffs des Patentanspruchs 1 bzw. 3, zur Gewebeablation ohne offene Operation.

Mit bildgebenden medizinischen Diagnoseverfahren wie Magnetresonanztomographie, Computertomographie, Ultraschall und dgl. ist es nicht nur möglich, die Lage, die Größe und die Form pathologischer Veränderungen im Körper aufzuzeigen, sondern auch mit Nadeln, Sonden und Kathetern durchgeführte Eingriffe ohne offene Operation zu verfolgen. Ein zunehmend erfolgreich ausgeführter Eingriff dieser Art ist die Gewebeablation, die vorwiegend zur Beseitigung von schlecht zugänglichem Gewebe dient, das heißt, der Abtötung bestimmter Gewebebereiche mit nachfolgender langsamer Resorption durch das umgebende Gewebe.

Neben der mechanischen Abtragung, bei der die Menge des abgetragenen Gewebes durch das kleinlumige Kaliber der medizinischen Instrumente begrenzt ist, ist auch die Zuführung von eine Gewebeablation bewirkenden Flüssigkeiten vorgesehen, was jedoch insofern nachteilig ist, als solche schädigenden Flüssigkeiten auch unkontrolliert in gesundes Gewebe abfließen und dieses schädigen können.

Schließlich kann mit den bekannten Geräten für einen kleinlumigen Gewebezugang auch durch Erzeugung von Wärmeenergie oder Kälte ein Abtöten von Gewebe bewirkt werden. Während die Gefrierablation aufgrund der dafür erforderlichen kräftigen Sonden und des dadurch bedingten großen Durchmessers von Kanülen und Kathetern nur in beschränktem Umfang einsetzbar ist, besteht der Nachteil der Hitzeablation bei Anwendung der Radiofrequenzmethode darin, dass dadurch die Bildgebung mittels Magnetresonanztomographie, mit der bei der Steuerung des Ablationsprozesses sehr gute Ergebnisse erzielt werden, gestört wird.

Als vorteilhaft hat sich in bezug auf die mit Hilfe der Magnetresonanztomographie durchgeführte Verfolgung und Steuerung der Hitzeablation, und zwar zur restlosen Abtötung des betreffenden Gewebebereiches ohne Schädigung gesunden Gewebes, die Anwendung von Laserlicht als Energiequelle erwiesen.

Folgende Probleme sind jedoch bei Verwendung dieses Verfahrens mit den jeweiligen Gewebezugangssystemen aufgetreten: Einfache Schaffung des Zugangs zum Gewebe ohne unnötige Schädigung oder Verletzung des Patienten, die mechanische und thermische Empfindlichkeit des Streukörpers (energieabgebende Zone) am distalen Ende des Laserlichtleiters, die Gefahr der Verkohlung des Gewebes in unmittelbarer Umgebung des Streukörpers, die exakte Positionierung des Ablators im Tumorgewebe, die Beobachtung und exakte Kontrolle des Ablationsprozesses im Hinblick auf die Vollständigkeit der Ablation und die Schonung des gesunden Gewebes.

Der Zugang zum Gewebe ist insbesondere bei empfindlichem Gewebe wie dem Zentralnervensystem oder auch im Fall der Lunge problematisch. Der Zerbrechlichkeit des Streukörpers muss mit Maßnahmen zu deren Schutz und zur Vermeidung unnötiger mechanischer Belastung begegnet werden. Die Verkohlung des Gewebes kann durch wirkungsvolle Ableitung der Wärme aus der unmittelbaren Nähe des Streukörpers vermieden werden. Schließlich soll einer Störung der bildgebundenen Diagnostik durch geeignete Verfahren und Materialien begegnet werden.

Ein entsprechendes Laserapplikationsgerät ist beispielsweise in der DE 196 14 780 A1 beschrieben. Es umfasst eine zunächst in den Körper einzuführende Punktionsnadel, durch deren hohlen Schaft nach Entfernen eines Mandrins ein Führungsdraht eingeführt wird. Nach dem Abziehen der Punktionsnadel wird über den Führungsdraht eine Einführschleuse mit in deren hohlen Schaft aufgenommenen hohlen Innenmandrin eingebracht. Anschließend werden der Führungsdraht und der hohle Mandrin entfernt. Die am distalen Ende offene Einführschleuse weist am proximalen Ende ein T-Stück auf, über das Lokalanästhetika, Gleitmittel oder Gewebekleber zugeführt werden können. Des weiteren umfasst dieses Laserapplikationssystem einen distal geschlossenen Hüllkatheter, der nach dem Herausziehen seines Innenmandrins einen Lichtwellenleiter für das Laserlicht aufnimmt.

Das aus einer Mehrzahl von Einzelteilen bestehende Gerät ist aufwendig ausgebildet und für den Zugang zum Gewebe ist dementsprechend eine Vielzahl von Schritten erforderlich. Außerdem wird der zu behandelnde Patient auch dadurch erheblich belastet, dass der Durchmesser der durch die Haut in den betreffenden Gewebebereich einzubringenden Einführschleuse relativ groß ist. Zwar ist der Streukörper, das heißt, die energieabgebende Zone des Lichtwellenleiters, durch den Hohlkatheter vor mechanischer Belastung und Zerstörung geschützt, jedoch kann der Laser nur mit geringer Leistung (bis ca. 6 Watt) betrieben werden. Bei höherer Leistung kommt es zu einer Verkohlung und Zerstörung des Systems durch ein zu konzentriertes Wärmeangebot in unmittelbarer Nähe der Spitze des Hüllkatheters.

Eine zur Vermeidung dieses Nachteils gemäß der DE 197 02 898 A1 vorgesehene Kühlung mit Hilfe eines weiteren Hüllkatheters erlaubt zwar mit bis zu 30 Watt eine relativ hohe Laserleistung, hat aber den Nachteil, dass die unmittelbar an dem Streukörper des Laserlichtleiters vorbeigeführte und wieder nach außen abgeleitete Kühlflüssigkeit einen erheblichen Teil der Wärmeleistung aus dem Gewebe abführt, der somit nicht für die Behandlung zur Verfügung steht. Zudem sind solche Systeme wegen der großen Kühlflüssigkeitsmengen (60 ml/min) und der damit verbundenen großen Querschnitte der Sonden und Katheter für bestimmte Anwendungen, beispielsweise beim Zentralnervensystem und der Lunge, wegen der großlumigen Einstichöffnungen nicht geeignet oder mit Risiken und Nachteilen für den Patienten verbunden.

Die WO 94/26184 löst das Problem der Verteilung der Lichtenergie bzw. Wärme im Gewebe durch die Injektion von Flüssigkeitsdepots, die das Licht streuen ohne es stark zu absorbieren. Die dafür vorgesehenen Katheter werden nach vorheriger Sondierung des zu verödenden Gewebeareals mit einer Sondiernadel und Erweiterung des Stichkanals eingeführt. Eine direkte Punktion des Gewebes mit der doppelumigen oder distal ganz oder weitgehend geschlossenen Kanüle ist nicht vorgesehen.

In der US 5,222,953 werden Instrumente beschrieben, die eine einfache direkte Punktion des Gewebes ermöglichen. Wegen der unabdingbaren Positionierung der Spitze des Lichtleiters unmittelbar am distalen Ende oder außerhalb der nicht oder nicht ausreichend lichtdurchlässigen Kanülen kann die energieabgebende zone nicht in dem notwendigen Maße gekühlt werden, so dass nur geringe Leistungen und damit Ablationsvolumina erreicht werden.

Der Erfindung liegt daher die Aufgabe zugrunde, eine durch bildgebende Verfahren kontrollierbare und steuerbare Vorrichtung zur Gewebeablation anzugeben, die einfach ausgebildet und zu handhaben ist und bei geringer Patientenbelastung hoch wirksam und vielseitig anwendbar ist.

Erfindungsgemäß wird die Aufgabe mit einem gemäß den Merkmalen der unabhängigen Ansprüche 1 und 3 ausgebildeten Gewebezugangs- und -behandlungssystem gelöst.

Aus den Unteransprüchen ergeben sich vorteilhafte Weiterbildungen der Erfindung.

Bei dem erfindungsgemäßen Gewebezugangs- und -behandlungssystem ist an einem den jeweiligen Energieleiter aufnehmenden, distal offenen, rohrförmigen Hohlkörper, und zwar in Form einer Kanüle oder eines Katheters, ein Griffstück angebracht, das mit einem axial mit dem Hohlorgan fluchtenden Durchgangskanal und einem an den Durchgangskanal angeschlossenen Infusionsanschlussstutzen versehen ist. Mit einer im Durchgangskanal angeordneten Dichtung wird ein luftdichter Abschluss zum betreffenden Gewebe sichergestellt. Über den Infusionsanschlussstutzen wird mit geringer Geschwindigkeit ein flüssiges oder gasförmiges Medium direkt oder mittelbar zur Kühlung über die energieabgebende Zone des Energieleiters geleitet und anschließend in das Gewebe infundiert. Damit steht die gesamte vom Energieleiter abgegebene Wärme für den Ablationsprozess zur Verfügung, während gleichzeitig eine Kühlung der energieabgebenden Zone erfolgt, so dass eine Schädigung des Gewebes durch Verkohlung aufgrund einer zu hohen wärmeabgabe ausgeschlossen ist. Das in das Gewebe infundierende flüssige oder gasförmige Medium kann außerdem eine den Ablationsprozess fördernde toxische oder therapeutische Wirkung haben, Schmerzen lindern und/oder zusätzlich als Kontrastmittel zur exakten Kontrolle und Steuerung des Prozesses mit Hilfe bildgebender Verfahren fungieren.

Die mit dem erfindungsgemäßen Gerät durchgeführte Gewebeabtötung ist daher vollständig und wird dennoch, insbesondere im Hinblick auf benachbartes gesundes Gewebe, schonend durchgeführt. Ein weiterer wesentlicher Vorteil besteht darin, dass das Hohlorgan in den erfindungsgemäßen Ausführungsvarianten beim Zuführen zu dem zu behandelnden Gewebeabschnitt und bei der Behandlung so nach außen abgedichtet werden kann, dass ein Lufteintritt mit seinen schädigenden Folgen für den Patienten ausgeschlossen werden kann. Zudem sind lediglich kleinlumige Einstichöffnungen erforderlich. Die energieabgebende Zone des Energieleiters ist geschützt untergebracht und kann daher nicht zerstört werden. Darüber hinaus ist die aus einer geringen Zahl von Einzelteilen bestehende Vorrichtung auch leicht zu handhaben. Außerdem ist der Gewebezugang z. B. auch für Biopsien ohne Wiederholung oder Austausch mit dem Körper in Kontakt befindlicher Teile mit einer einzigen Punktion möglich. Das erfindungsgemäße Gerät ist daher effektiv und dennoch ohne Risiken und Nachteile für die zu behandelnden Personen einsetzbar.

Als mit der erfindungsgemäßen Vorrichtung infundierte Mittel zur Verstärkung der Gewebeablation werden vorzugsweise Ethanol, Essigsäure oder in Wasser verdünnte Essigsäure, Dimethylsulfoxid, das gleichzeitig der verbesserung der Lichttransmission dient, oder Dimethylformamid eingesetzt.

Für therapeutische Zwecke können Anästhetika zur lokalen Schmerzlinderung bzw. Zytostatika, Radiopharmaka oder andere Tumortherapeutika zur Vermeidung lokaler und regionaler Tumorrezidive direkt in das betroffene Gewebe infiltriert werden.
Vorzugsweise infundierte Kontrastmittel sind jodierte Verbindungen, paramagnetische oder superparamagnetische Verbindungen, oder Mittel mit feinen Gasbläschen, wie sie bei Röntgenverfahren bzw. bei der Magnetresonanztomagraphie bzw. in der Ultraschalldiagnostik angewandt werden.

Als Gase zur Verstärkung des Ablationsvorganges werden Kohlenmonoxid und ähnlich wirkende toxische Gase eingesetzt. Des weiteren können medizinische Gase wie beispielsweise Kohlendioxid, Sauerstoff, Xenon oder Narkosegase über die Kanüle bzw. den distal offenen Katheter zugeführt werden.

Die Dosierung der nekroseverstärkenden Mittel beträgt 0,1 bis 5 ml/min während und 0,5 bis 50 ml/min vor bzw. nach der thermischen Gewebeablation.

In einer ersten Ausführungsvariante ist das erfindungsgemäß ausgebildete röhrenförmige Hohlorgan eine Kunststoffkanüle, in der der Energieleiter unmittelbar angeordnet und seine energieabgebende Zone direkt von dem zugeführten Medium umspült wird. In diese Kanüle mit Medienanschluss kann aber auch ein den Energieleiter aufnehmender distal geschlossener oder offener seinerseits durchspülbarer Katheter eingebracht werden. Dessen Außendurchmesser muss jedoch deutlich kleiner als der Innendurchmesser der Kanüle sein, um den erforderlichen Strömungsquerschnitt für das der Kanüle zugeführte Medium zu schaffen. Im Falle des geschlossenen Katheters wird die energieabgebende Zone, die in der Kanüle bleiben muss, nur indirekt gekühlt.

Gemäß der Erfindung ist die energieabgebende Zone des Energieleiters in einem die Energie- oder Wärmewirkung auf das Gewebe nicht behindernden Bereich des Hohlorgans bzw. der Hohlorgane angeordnet, das heißt, der Wärmewirkung bzw. Energieabgabe steht kein für die von dem Energieleiter abgegebene Energieart undurchlässiges Material der Kanüle oder des Katheters entgegen. Die Energieübertragung kann auch durch Perforation der Kanülen- oder Katheterwand erreicht werden. Vorzugsweise sind die Kanülen und Katheter zumindest im Bereich der energieabgebenden Zone des Energieleiters in dem zu erwartenden Temperaturbereich hitzebeständig und soweit energiedurchlässig, dass mindestens 50 % der vom Energieleiter abgegebenen Energie in das Gewebe übertreten können. Bei Verwendung einer nicht oder wenig energiedurchlässigen Kanüle ist das die energieabgebende Zone des Energieleiters schützende Kanülenende so positioniert, dass die Kanüle der Energieabgabe in das Gewebe nicht entgegensteht.

In einer weiteren Ausführungsvariante stellt das erfindungsgemäß ausgebildete Hohlorgan einen den Energieleiter direkt aufnehmenden Katheter dar, der in eine einfach ausgebildete Kanüle mit am proximalen Ende angeordneter Dichtung eingebracht wird. In diesem Fall wird die energieabgebende Zone des Energieleiters innerhalb des Katheters direkt mit dem zugeführten Medium beaufschlagt.

Als Kanülen- und Kathetermaterial werden energie- und lichtdurchlässige Kunststoffe, wie beispielsweise PTFE (Teflon), PEEK, (Polyether-Etherketon) oder Polyimid eingesetzt, die auch bei geringen Wandstärken im Bereich von 0,02 - 0,5 mm und in einem Durchmesserbereich von 2 mm und weniger noch eine ausreichende Festigkeit aufweisen. Für die Kanülen können darüber hinaus auch nicht ferromagnetische Metalle, wie beispielsweise Titan, verwendet werden, wobei in diesem Falle das distale Ende des Katheters zur Gewährleistung der Energieabgabe in das Gewebe entsprechend zu positionieren ist.

In Ausgestaltung der Erfindung ist am Außenumfang der Kanüle ein verstellbarer Anschlag zur Begrenzung der Einstichtiefe, und zwar beim Einbringen der Kanüle oder auch bei Bewegungen des Patienten während der Behandlung, angeordnet.

Nach einem weiteren Merkmal der Erfindung befindet sich in der jeweiligen Kanüle ein vorzugsweise viskoses Dichtungsmittel, um das Eindringen von Luft zu verhindern.

Ausführungsbeispiele der Erfindung werden anhand der Zeichnung, in der die Vorrichtung zur Gewebeablation der Anschaulichkeit halber in vergrößertem Maßstab wiedergegeben ist, näher erläutert. Es zeigen:
- Fig. 1: einen Mandrin mit proximal angebrachtem Griff
- Fig. 2: eine den Mandrin nach Fig. 1 aufnehmende und von diesem ausgefüllte, beidseitig offene Kanüle mit einem proximal an dieser angebrachten Griffstück mit Infusionsanschlussstutzen und einem abdichtbaren axialen Durchgangskanal;
- Fig. 3: einen distal geschlossenen Katheter mit Griffstück, in dessen axialen Durchgangskanal eine Dichtung vorgesehen ist;
- Fig. 4: einen weiteren Mandrin mit proximal angeordnetem Griff für eine andere, in Fig. 5 dargestellte Kanüle;
- Fig. 5: eine Kanüle mit proximal angeordnetem Griff, in dem ein mit der Kanüle fluchtender Durchgangskanal ausgebildet ist;
- Fig. 6: einen distal offenen Katheter mit proximal angeordnetem Griffstück, das einen seitlichen Infusionsanschlussstutzen zur Gas- oder Flüssigkeitszufuhr und einen axialen abdichtbaren Durchgangskanal aufweist;
- Fig. 7: die Kanüle nach Fig. 2 mit dem in diese eingeführten, distal geschlossenen Katheter nach Fig. 3, in den der Energieleiter einführbar ist;
- Fig. 8: die Kanüle nach Fig. 5 mit dem in diese eingeführten Katheter nach Fig. 6, wobei das flüssige oder gasförmige Medium unmittelbar über die energieabgebende Zone eines in dem Katheter zu positionierenden Energieleiters zum Gewebe geleitet wird und die Positionierung des Katheters mittels einer an diesem vorgesehenen Markierung angezeigt wird.

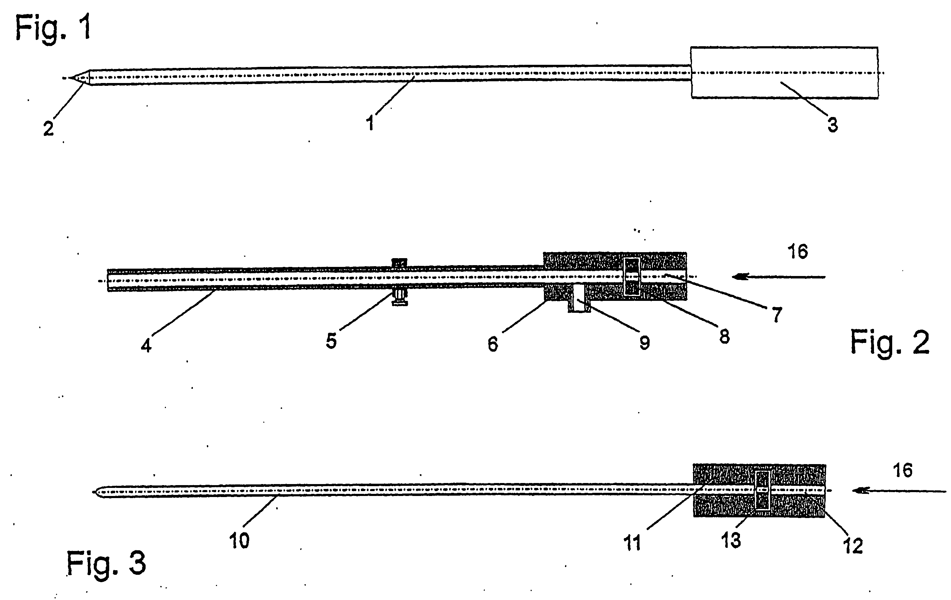

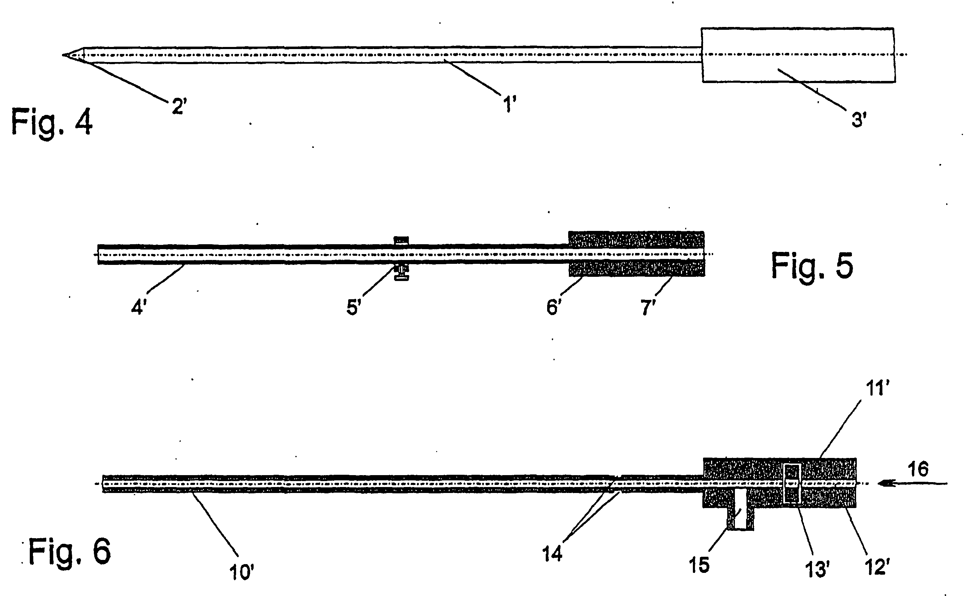

Der Mandrin 1, 1' gemäß den Figuren 1 und 4 ist schwer verbiegbar und weist am distalen Ende eine Spitze 2, 2' und am proximalen Ende einen kurzen Griff 3, 3' auf. Der Mandrin 1 ist geringfügig länger als die jeweilige Kanüle 4, 4' gemäß den Figuren 2 und 5, so dass seine Spitze 2, 2' aus der distalen Öffnung der Kanüle, in die er gasdicht eingepasst sein kann, herausragt. Sofern das energieabgebende System in der Kanüle verbleibt, bestehen die Kanülen 4, 4' am distalen Ende oder vollständig aus für die vom Energieleiter abgegebene Energie durchlässigem, hitzebeständigem Material. Am Außenumfang der Kanüle 4, 4' ist ein verstellbarer Anschlag 5 zur Begrenzung der Einstichtiefe angebracht.

In einer ersten Ausführungsvariante gemäß den Figuren 1 und 2 wird die Kanüle 4 in einer zumindest distal energiedurchlässigen Ausführung mit dem in deren rohrförmigem Hohlraum liegenden Mandrin 1 in das abzutötende Gewebe eingeführt. Durch Füllen des Hohlraumes der Kanüle 4 mit einem sterilen, pharmazeutischen Öl kann unabhängig von der Dichtung 8 ein nach außen luftdichter Abschluß erzeugt werden, so dass beispielsweise bei einer in der Lunge durchzuführenden Gewebeablation keine Luft in den Thoraxraum eindringen kann. Um den Lufteintritt in den Pleuraspalt mit Sicherheit auszuschließen, wird der Mandrin 1 erst aus der Kanüle 4 herausgezogen, wenn das distale Ende der Kanüle 4 im betreffenden Gewebe platziert ist. Nach der Entnahme des Mandrins 1 wird beispielsweise ein handelsüblicher Laserlichtleiter (Energieleiter) 16 mit distal angeordnetem Streukörper (energieabgebende Zone) (nicht dargestellt) über den axialen Durchgangskanal 7 eines an der Kanüle 4 angebrachten Griffstückes 6 so weit in die Kanüle 4 eingeführt, dass sich der empfindliche Streukörper proximal wenige Millimeter vor der Kanülenöffnung befindet und somit vor Zerstörung geschützt ist. Der Laserlichtleiter 16 wird in dem Durchgangskanal 7 des Griffstückes 6 mit Hilfe eines Dichtringes 8 (oder einer nicht dargestellten Stopfbuchse) in der Arbeitsstellung fixiert und nach außen abgedichtet.

Über einen seitlich in das Griffstück 6 integrierten Infusionsanschlussstutzen 9, der in den Durchgangskanal 7 mündet, wird der Streukörper mit einer Infusionsgeschwindigkeit von 0,1 bis 2 ml/min mit einer Kühlflüssigkeit beaufschlagt. Dadurch wird eine Verkohlung des Gewebes in unmittelbarer Nähe des Streukörpers durch zu hohe wärmewirkung vermieden. Andererseits wird die gleichzeitig als Wärmetransportmittel dienende Kühlflüssigkeit aber auch zum Gewebe weitergeleitet, so dass die vom Streukörper abgegebene Wärme dennoch an das Gewebe gelangt und nicht verloren geht. Die Kühlflüssigkeit kann gleichzeitig als Ablationsmittel und/oder als Kontrastmittel ausgebildet sein. Anstelle einer Flüssigkeit kann auch Gas in die Kanüle geleitet werden, wobei jedoch die Kühlwirkung entfällt.

Die Gewebeablation kann, wie die Figuren 3 und 6 bis 8 zeigen, des weiteren auch mit einem Laserlichtleiter, der sich in einem zusätzlich in die Kanülen 4, 4' eingebrachten Katheter 10, 10' befindet, durchgeführt werden.

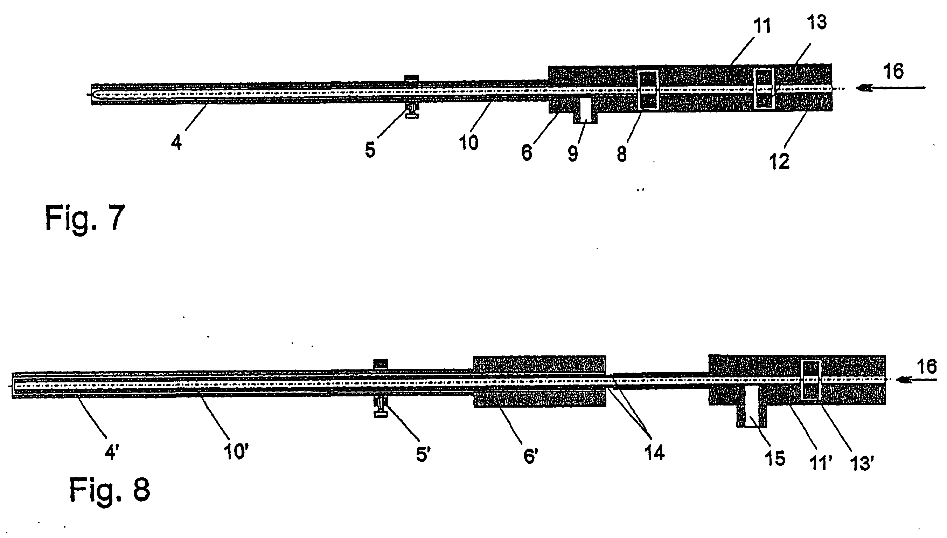

Der Katheter 10 kann, wie Fig. 3 zeigt, distal geschlossen sein und proximal mit einem Griffstück 11 mit axialem Durchgangskanal 12 und einer Dichtung 13 versehen sein. Dieser Katheter 10 wird, nachdem der zugehörige Mandrin 1 nach dem Einführen der Kanüle 4 in das betreffende Gewebe wieder herausgezogen wurde, in die Kanüle 4 eingeschoben. Der Außendurchmesser des Katheters 10 ist kleiner als der Innendurchmesser der Kanüle 4. Zwischen der Kanüle 4 und dem Katheter 10 wird daher ein Hohlraum mit kreisringförmigem Querschnitt gebildet. Der Streukörper eines gemäß Fig. 7 in den Katheter 10 einzuführenden Laserlichtleiters 16 ist in dem geschlossenen Katheter 10 geschützt untergebracht und über die in dessen Griffstück 11 angeordnete Dichtung 13 fixiert und nach außen abgedichtet. Der in die Kanüle 4 eingeführte Katheter 10 ist seinerseits über den Dichtring 8 in dem Griffstück 6 nach außen abgedichtet und ragt in eingeführtem Zustand nicht über die distale Öffnung der Kanüle 4 hinaus. Ihr distales Ende besteht, wie das der Kanüle 4, ebenfalls aus für die vom Energieleiter abgegebene Energie durchlässigem, hitzebeständigem Material. Über den Infusionsanschlussstutzen 9 im Griffstück 6 sowie den kreisringförmigen Hohlraum zwischen Katheter und Kanüle wird in der oben beschriebenen Weise wieder Kühlflüssigkeit und gegebenenfalls Ablations- und/oder Kontrastflüssigkeit mittelbar zum Streukörper und direkt zu dem betreffenden Gewebeabschnitt geleitet.

Eine weitere, aus Fig. 8 ersichtliche Ausführungsvariante der Erfindung ergibt sich aus einer Kombination des in Fig. 4 gezeigten Mandrin 1 mit der in Fig. 5 dargestellten Kanüle 4', in die nach dem Einbringen in das Gewebe und nach dem Entfernen des Mandrins 1 ein Katheter 10' nach Fig. 6 eingesteckt wird. Die Kanüle 4' weist proximal ein Griffstück 6' mit Durchgangskanal 7' und einen verstellbaren Anschlag 5' auf. Der Katheter 10' ist länger als die Kanüle 4', weist jedoch eine Markierung 14 auf, bei der die beiden distalen Enden der Kanüle und des Katheters zueinander bündig abschließen. Die Abdichtung und feste Positionierung des Katheters 10' in der Kanüle 4' erfolgt mit einer Stopfbuchse (nicht dargestellt). In diesem Fall ist der Katheter 10' proximal mit einem Griffstück 11' versehen, das wie das Griffstück 6 der in Fig. 2 dargestellten Kanüle 4 ausgebildet ist und einen Durchgangskanal 12' mit einer Dichtung 13' sowie einen Infusionsanschlussstutzen 15 zur unmittelbaren Zufuhr eines Kühl-, Ablations- und/oder Kontrastmittels in flüssigem oder gasförmigem Zustand unmittelbar zum Streukörper (energieabgebende Zone) eines in den Katheter 10' eingeführten Laserlichtleiters (Energieleiter) und zu dem behandelten Gewebe aufweist. Der Anschlussstutzen 15 (9) ist vorzugsweise als Luer-Lock-Anschluß ausgebildet, während als Dichtung (8, 13, 13') ein Dichtring mit Quetschverschraubung vorgesehen ist. Vor der Aktivierung des Energieleiters 16 wird die Kanüle 4' bis zum Anschlag am Griffstück 11' aus dem Gewebe zurückgezogen, so dass das distale Ende des Katheters 10' über die Länge der energieabgebenden Zone des Energieleiters aus der Kanüle 4' herausragt und somit die Energieabgabe in das Gewebe nicht behindert wird.

Das erfindungsgemäße Gewebezugangssystem soll im Folgenden durch vergleichende Versuche am Objekt Rinderleber verdeutlicht werden. Verglichen wird die Leistungsfähigkeit des erfindungsgemäßen Prinzips mit dem Stand der Technik (DE 197 02 898 A1).

Die durch die Anwendung von Laserlicht hoher Energie bedingte Leistung der Systeme besteht in der Erhitzung von Gewebe, die u.a. zu einer Eiweißgerinnung führt. Die Größe der erhitzten Gewebezone wird einerseits durch die Leistung des Laserlichts (Wattzahl) und andererseits durch die unerwünschte Verkohlung von Gewebe in unmittelbarer Nähe der energieabgebenden Zone des Lichtleiters und schließlich die Zerstörung dieser Zone durch Überhitzung bedingt. Um Überhitzung und Verkohlung zu vermeiden, wird die energieabgebende Zone der Lichtleiter gekühlt. Der Stand der Technik wird durch DE 197 002 898 A1 repräsentiert. Im Folgenden wird daher ein Vergleich zu den Systemen mit Kühlmittelzufluß und Abfluß herangezogen.

### Typische Anwendung (Leistungsgrenze) :

| | DE 197 02 898 A1 | Erfindung |
|---|---|---|
| Bezeichnung | Somatex Power Applikator | Mikrokath |
| Watt (max.) | 25 | 15 |
| Kühlung, ml/min | 60 | 0,75 |
| verbleib der Kühlflüssigkeit | Rückführung | Im Gewebe |

Dazu wurden in üblicher Weise Vergleichsversuche an frischer Rinderleber mit beiden Systemen durchgeführt, wobei die erhitzte Zone nach Aufschneiden des Gewebes durch helle Verfärbung (Eiweißgerinnung) und die an sich ungefährliche, aber unerwünschte Überhitzung durch Schwarzfärbung gut erkenntlich wird.

### Materialien:

1.) Somatex Power-Laserapplikationsset, durchstromgekühlt nach Herstellerangabe mit 60 ml/min, Außendurchmesser ca. 3 mm.
2.) Mikrokath nach DPA 101 01 143.1, infusionsgekühlt, Außendurchmesser 1,37 mm (Versuch Nr. 1-3) bzw. 1,80 mm (Versuch Nr. 4-6).
3.) Für beide Systeme Hüttinger GmbH, Umkirch, Mikrodom Applikator A13-0540 bzw. Dornier Medizin Laser GmbH, Germering, H-6111-T3 Diffusor Tip Lichtleiter angeschlossen an Dornier Fibertom.

### Durchführung:

Die jeweiligen Applikatorsysteme wurden tief in der Rinderleber platziert, der Lichtleiter eingeführt, die Laserung unter den beschriebenen Bedingungen durchgeführt und die Leber im Anschluss daran parallel zur Lichtleiterführung aufgeschnitten und die durch Eiweißgerinnung hell verfärbte Zone vermessen. Die mit "P1" bis "P6" bezeichneten Versuche (siehe Tabelle 1) betreffen die Behandlung mit dem Somatex-Power-Applikatorset, die mit "1" bis "6" bezeichneten Versuche (siehe Tabelle 1) die Behandlungen mit dem Mikrokath. Aus der langen und kurzen Achse der hell verfärbten Gerinnungs-Ellipsoide wurde das Koagulationsvolumen berechnet. Die zentrale Zone zeigt häufig eine unerwünschte Schwärzung (Karbonisierung), die die weitere Hitzeausbreitung behindert.

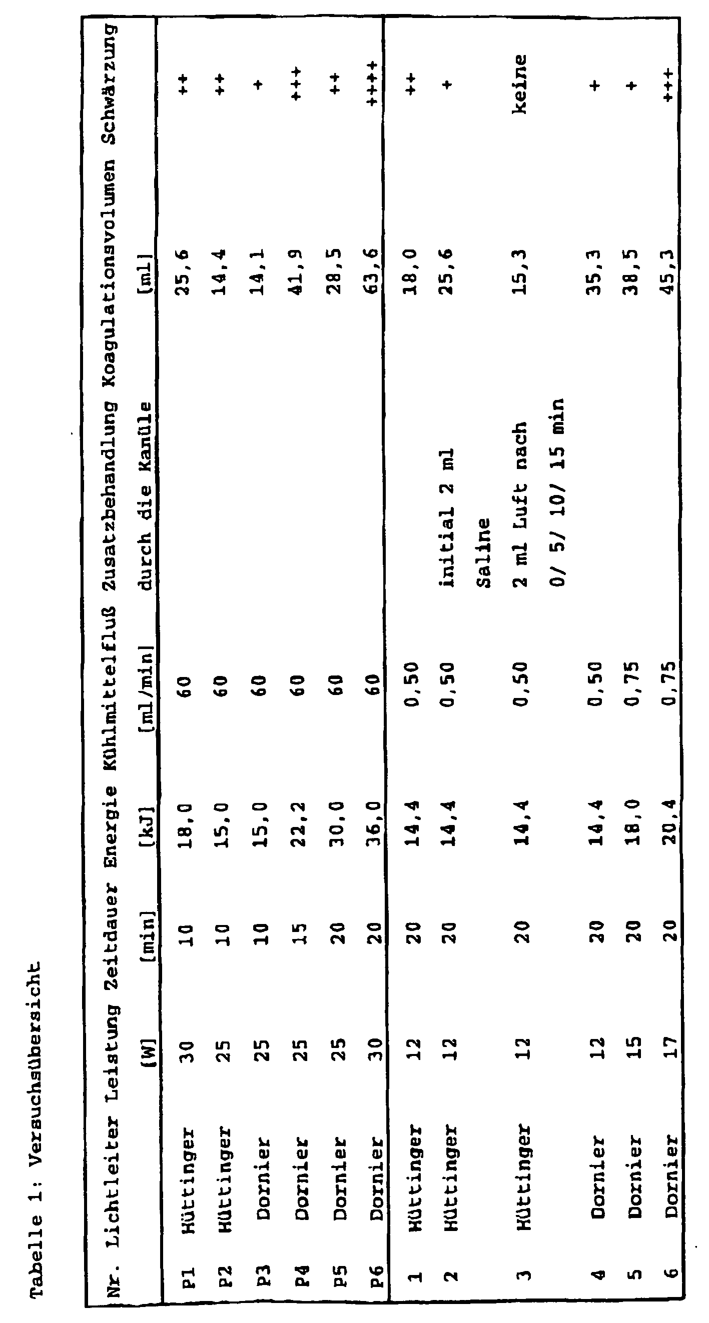

### Ergebnis und Schlussfolgerung:

Überraschenderweise werden trotz der geringen Abmessungen des Mikrokath und der wesentlich geringeren Laserleistung mit dem Mikrokath ähnlich große Koagulationszonen erreicht wie mit dem Somatex Power Applikator. Vermutlich wird durch den intensiven Kühlflüssigkeitsstrom des Somatex-Systems ein Teil der Energie aus dem Gewebe herausgetragen. Ebenso erstaunlich ist, dass es bei Anwendung des Mikrokath trotz des auf weniger als 2 % reduzierten Kühlflüssigkeitsstroms zu keiner stärkeren zentralen Schwärzung des Gewebes kommt. Bei gleicher Leistungsfähigkeit ist das erfindungsgemäße System wesentlich einfacher aufgebaut, einfacher anwendbar, hinterlässt geringe Stichkanalschäden und ist zusätzlich für Lungenmetastasen geeignet.

### Bezugszeichenliste

- 1, 1': Mandrin
- 2, 2': Spitze von 1, 1'
- 3, 3': Griff
- 4, 4': Kanüle
- 5, 5': verstellbarer Anschlag
- 6, 6': Griffstück von 4, 4'
- 7, 7': Durchgangskanal von 6,6'
- 8: Dichtung von 6
- 9: Infusionsanschlussstutzen v. 6
- 10, 10': Katheter
- 11, 11': Griffstück von 10, 10'
- 12, 12': Durchgangskanal von 11, 11'
- 13, 13': Dichtung von 11
- 14: Markierung
- 15: Infusionsanschlussstutzen von 11'
- 16: Energieleiter (Laserlichtleiter)

## Patentansprüche

1. Steriles Gewebezugangs- und -behandlungssystem, das folgende Teile umfasst:
a) eine aus nicht ferromagnetischem, hitzebeständigem Material bestehende, distal offene einlumige Kanüle (4) mit proximal vorgesehenem Griffstück (6) und darin verlaufendem Durchgangskanal (7), welches einen Infusionsanschlussstutzen (9) zur Medienzufuhr zum Gewebe aufweist;
b) einen Mandrin (1) zum Einführen in die Kanüle (4), wobei für den Gewebezugang der Mandrin (1) aus der Kanüle (4) herausragt;
c) einen Energieleiter (16) zum Einführen in die Kanüle (4) mit an dessen freiem Ende vorgesehener energieabgebender Zone,
wobei, wenn der Energieleiter (16) in die Kanüle (4) eingeführt ist, ein ringförmiger Strömungskanal zwischen dem Energieleiter (16) und der Kanüle (4) zur Medienzufuhr zum Gewebe entsteht, **dadurch gekennzeichnet, dass**
- in den Durchgangskanal (7) ein einstellbarer Dichtring (8) mit Quetschverschraubung zur gasdichten Fixierung des Energieleiters (16) und des Mandrins (1) integriert ist; und
- die Kanüle (4) für die vom Energieleiter (16) abgegebene Energie durchlässig ist, so dass der Energieleiter (16) während der Behandlung innerhalb der Kanüle angeordnet sein kann.

2. Gewebezugangs- und -behandlungssystem nach Anspruch 1, **dadurch gekennzeichnet, dass** der Energieleiter (16) in einem distal geschlossenem Katheter (10) mit proximal angeschlossenem Griffstück (11), in dessen Durchgangskanal (12) eine Dichtung (13) zur Abdichtung und Fixierung des Energieleiters (16) vorgesehen ist, angeordnet ist, wobei der ringförmige Strömungskanal zwischen Katheter (10) und Kanüle (4) gebildet ist, wenn sich der Katheter (10) mit Energieleiter (16) in der Kanüle (4) befindet, und das distale Ende des Katheters (10) nicht über das der Kanüle (4) hinausragt.

3. Steriles Gewebezugangs- und -behandlungssystem, das folgende Teile umfasst:
a) einen aus nicht ferromagnetischem, hitzebeständigem Material bestehenden, distal offenen Katheter (10') mit proximal vorgesehenem Griffstück (11') und darin verlaufendem Durchgangskanal (12'), welcher einen Infusionsanschlussstutzen (15) zur Medienzufuhr zum Gewebe aufweist;
b) einen Energieleiter (16) zum Einführen in den Katheter (10') mit an dessen freiem Ende vorgesehener energieabgebender Zone,
wobei, wenn der Energieleiter (16) in den Katheter (10') eingeführt ist, ein ringförmiger Strömungskanal zwischen dem Energieleiter (16) und dem Katheter (10') zur Medienzufuhr zum Gewebe entsteht, **dadurch gekennzeichnet, dass**
- das System weiterhin eine einlumige Kanüle (4') mit proximal vorgesehenem Griffstück (6') und darin verlaufendem Durchgangskanal (7') umfasst, worin der Katheter (10') eingeführt werden kann;
- das System weiterhin einen Mandrin (1') zum Einführen in die Kanüle (4') umfasst, wobei für den Gewebezugang der Mandrin (1') aus der Kanüle (4') herausragt;
- in den Durchgangskanal (12') des Katheters (10') ein einstellbarer Dichtring (13') mit Quetschverschraubung zur gasdichten Fixierung des Energieleiters (16) integriert ist; und
- der Katheter (10') für die vom Energieleiter (16) abgegebene Energie durchlässig ist, so dass der Energieleiter während der Behandlung innerhalb des Katheters (10') angeordnet sein kann.

4. Gewebezugangs- und -behandlungssystem nach Anspruch 3, **dadurch gekennzeichnet, dass** der Katheter (10') länger als die Kanüle (4') ist und eine Markierung (14) zur Positionierung seines distalen Endes in Bezug auf das distale Ende der Kanüle (4') aufweist.

5. Gewebezugangs- und -behandlungssytem nach Anspruch 2 oder 3, **dadurch gekennzeichnet, dass** der Katheter (10, 10') mindestens im Bereich der energieabgebenden Zone des Energieleiters (16) aus hitzebeständigem und für die vom Energieleiter (16) abgegebene Energie durchlässigem Material besteht.

6. Gewebezugangs- und -behandlungssystem nach einem der Ansprüche 1 bis 5, **dadurch gekennzeichnet, dass** der Energieleiter (16) einen Streukörper an seinem energieabgebenden distalen Ende aufweist.

7. Gewebezugangs- und -behandlungssystem nach einem der Ansprüche 1 bis 6, **dadurch gekennzeichnet, dass** an der Kanüle (4, 4') ein in Längsrichtung verstellbarer Anschlag (5, 5') zur Einstichtiefenbegrenzung angebracht ist.

8. Gewebezugangs- und -behandlungssystem nach einem der Ansprüche 1 bis 7, **dadurch gekennzeichnet, dass** zur Verhinderung des Eindringens von Luft in das zu behandelnde Organ in den verbleibenden engsten Spalten zwischen der Kanüle (4, 4') und dem Mandrin (1) eine sterile pharmazeutische Dichtflüssigkeit vorhanden ist.

9. Gewebezugangs- und -behandlungssystem nach einem der Ansprüche 1 bis 8, **dadurch gekennzeichnet, dass** das energiedurchlässige Kanülen- und Kathetermaterial für Schall sowie langwelliges als auch infrarotes Licht durchlässig ist.

10. Gewebezugangs- und -behandlungssystem nach einem der Ansprüche 1 bis 9, **dadurch gekennzeichnet, dass** der Energieleiter (16) an eine Laserlichtquelle oder eine Infrarot-Strahlungsquelle oder eine Kurzwellenschallquelle oder eine Dampfquelle angeschlossen ist.

11. Gewebezugangssystem nach einem der Ansprüche 1 bis 10, **dadurch gekennzeichnet, dass** der Infusionsanschlussstutzen (9, 15) an ein gasförmiges oder flüssiges Medienreservoir angeschlossen ist, das als Dichtmittel oder Kühl- und Wärmetransportmittel oder Kontrastmittel oder Ablationsmittel oder Therapeutikum dient.

12. Gewebezugangs- und -behandlungssystem nach einem der Ansprüche 1 bis 3, **dadurch gekennzeichnet, dass** das Kühlmedium mit einer Geschwindigkeit von 0,1 bis 2 ml/Minute durch den ringförmigen Strömungskanal strömt.

## Revendications

1. Système stérile d'accès à des tissus et de traitement de ces tissus, qui comprend les éléments suivants :
a) une canule (4) à lumen unique, ouverte côté distal et constituée par un matériau non ferromagnétique thermorésistant, comportant un élément de poignée (6) prévu côté proximal et un canal traversant (7) s'étendant dans celui-ci, lequel présente un manchon de raccordement à perfusion (9) pour l'amenée de substances aux tissus ;
b) un mandrin (1) destiné à être introduit dans la canule (4), le mandrin (1) faisant saillie hors de la canule (4) pour l'accès aux tissus ;
c) un conducteur d'énergie (16) destiné à être introduit dans la canule (4), avec une zone fournissant de l'énergie prévue à son extrémité libre, dans lequel, lorsque le conducteur d'énergie (16) est introduit dans la canule (4), il est formé un canal d'écoulement annulaire entre le conducteur d'énergie (16) et la canule (4) pour l'amenée des substances aux tissus, **caractérisé en ce que**
- un joint d'étanchéité (8) réglable avec vissage à écrasement pour la fixation étanche aux gaz du conducteur d'énergie (16) et du mandrin (1) est intégré dans le canal traversant (7) ; et
- la canule (4) laisse passer l'énergie fournie par le conducteur d'énergie (16), de telle sorte que le conducteur d'énergie (16) peut être agencé à l'intérieur de la canule pendant le traitement.

2. Système stérile d'accès à des tissus et de traitement de ces tissus selon la revendication 1, **caractérisé en ce que** le conducteur d'énergie (16) est agencé dans un cathéter (10) fermé côté distal avec élément de poignée (11) raccordé côté proximal, dans le canal traversant (12) duquel est prévu un joint (13) pour étancher et fixer le conducteur d'énergie (16), dans lequel le canal d'écoulement annulaire est formé entre le cathéter (10) et la canule (4) lorsque le cathéter (10) se trouve avec le conducteur d'énergie dans la canule (4), et l'extrémité distale du cathéter (10) ne dépasse pas au-delà de la canule (4).

3. Système stérile d'accès à des tissus et de traitement de ces tissus, qui comprend les éléments suivants :
a) un cathéter (10') ouvert côté distal, constitué par un matériau non ferromagnétique et thermorésistant, comportant un élément de poignée (11') prévu côté proximal et un canal traversant (12') s'étendant dans celui-ci, lequel présente un manchon de raccordement à perfusion (15) pour l'amenée de substances aux tissus ;
b) un conducteur d'énergie (16) destiné à être introduit dans le cathéter (10'), avec une zone fournissant de l'énergie prévue à son extrémité libre,
dans lequel, lorsque le conducteur d'énergie (16) est introduit dans le cathéter (10'), il est formé un canal d'écoulement annulaire entre le conducteur d'énergie (16) et le cathéter (10') pour l'amenée des substances aux tissus, **caractérisé en ce que**
- le système comprend en outre une canule (4') à lumen unique avec un élément de poignée (6') prévu côté proximal et un canal traversant (7') s'étendant dans celui-ci et dans lequel le cathéter (10') peut être introduit ;
- le système comprend en outre un mandrin (1') destiné à être introduit dans la canule (4'), le mandrin (1') faisant saillie hors de la canule (4') pour l'accès aux tissus ;
- un joint d'étanchéité (13') réglable avec vissage à écrasement pour la fixation étanche aux gaz du conducteur d'énergie (16) est intégré dans le canal traversant (10') du cathéter (10') ; et
- le cathéter (10') laisse passer l'énergie fournie par le conducteur d'énergie (16), de telle sorte que le conducteur d'énergie (16) peut être agencé à l'intérieur du cathéter (10') pendant le traitement.

4. Système stérile d'accès à des tissus et de traitement de ces tissus selon la revendication 3, **caractérisé en ce que** le cathéter (10') est plus long que la canule (4') et présente une marque (14) pour positionner son extrémité distale par rapport à l'extrémité distale de la canule (4').

5. Système stérile d'accès à des tissus et de traitement de ces tissus selon la revendication 2 ou 3, **caractérisé en ce qu'**au moins dans la région de la zone fournissant de l'énergie, du conducteur d'énergie (16), le cathéter (10, 10') est constitué par un matériau thermorésistant et laissant passer l'énergie fournie par le conducteur d'énergie (16).

6. Système stérile d'accès à des tissus et de traitement de ces tissus selon l'une des revendications 1 à 5, **caractérisé en ce que** le conducteur d'énergie (16) présente un corps diffuseur à son extrémité distale fournissant de l'énergie.

7. Système stérile d'accès à des tissus et de traitement de ces tissus selon l'une des revendications 1 à 6, **caractérisé en ce que** sur la canule (4, 4') est prévue une butée (5, 5') réglable en direction longitudinale pour limiter la profondeur de piquage.

8. Système stérile d'accès à des tissus et de traitement de ces tissus selon l'une des revendications 1 à 7, **caractérisé en ce que** pour empêcher que de l'air pénètre dans l'organe à traiter, il est prévu un liquide d'étanchéité pharmaceutique stérile dans les fentes les plus étroites demeurant entre la canule (4, 4') et le mandrin (1).

9. Système stérile d'accès à des tissus et de traitement de ces tissus selon l'une des revendications 1 à 8, **caractérisé en ce que** le matériau de canule et de cathéter, qui laisse passer l'énergie, laisse aussi passer le son ainsi que la lumière à grande longueur d'onde ainsi que la lumière infrarouge.

10. Système stérile d'accès à des tissus et de traitement de ces tissus selon l'une des revendications 1 à 9, **caractérisé en ce que** le conducteur d'énergie (16) est branché à une source de lumière laser ou à une source de rayonnement infrarouge ou à une source sonore à ondes courtes ou à une source de vapeur.

11. Système stérile d'accès à des tissus et de traitement de ces tissus selon l'une des revendications 1 à 10, **caractérisé en ce que** le manchon de raccordement à perfusion (9, 15) est branché à un réservoir de substances gazeuses ou liquides, qui servent de substances d'étanchéité ou de substances de transport réfrigérant ou chauffant ou de substances de contraste ou d'ablation ou de produits thérapeutiques.

12. Système stérile d'accès à des tissus et de traitement de ces tissus selon l'une des revendications 1 à 3, **caractérisé en ce que** la substance réfrigérante s'écoule à travers le canal d'écoulement annulaire avec une vitesse de 0,1 à 2 ml/minute.

## Claims

1. A sterile tissue access and treatment system that comprises the following components:
a) a distally open single lumen needle (4) consisting of a non-ferromagnetic, heat-resistant material with a proximally mounted handle piece (6), a through channel (7) running through the latter and said handle piece comprising an infusion fitting (9) for supplying an infusion medium to the tissue;
b) a mandrin (1) for insertion into the needle (4), said mandrin (1) protruding from the needle (4) for tissue access;
c) an energy conductor (16) for insertion into the needle (4) with an energy-releasing zone provided at its free end;
where an annular flow duct is formed between the energy conductor (16) and needle (4) when said energy conductor (16) is inserted in said needle (4) to facilitate media supply to the tissue, **characterized in that**
- an adjustable sealing ring (18) with a screw-on crimped connection for gas-tight fixation of the energy conductor (16) and the mandrin (1) is integrated in the through channel (7); and
- the needle (4) is permeable to the energy released by the energy conductor (16) so that the energy conductor (16) can be located inside the needle during treatment.

2. The tissue access and treatment system according to claim 1, **characterized in that** the energy conductor (16) is provided in a distally closed catheter (10) with a proximally mounted handle piece (11), in the through channel (12) of which a seal (13) is provided for sealing off and fixing the energy conductor (16), wherein an annular flow duct is formed between the catheter (10) and the needle (4) when the catheter (10) with energy conductor (16) is located inside the needle (4) and the distal end of the catheter (10) does not protrude beyond the needle (4).

3. A sterile tissue access and treatment system that comprises the following components:
a) a distally open catheter (10') consisting of a non-ferromagnetic, heat-resistant material with a proximally mounted handle piece (11') comprising a through channel (12') running through it and an infusion fitting (15) for supplying an infusion medium to the tissue;
b) an energy conductor (16) for insertion into the catheter (10') with an energy-releasing zone provided at its free end;
where an annular flow duct is formed between the energy conductor (16) and catheter (10') when said energy conductor (16) is inserted in said catheter (10') to facilitate media supply to the tissue,
**characterized in that**
- the system further includes a single lumen needle (4') with proximally mounted handle piece (6') and a through channel (7') running through it into which the catheter (10') can be inserted;
- the system further includes a mandrin (1') for insertion into the needle (4'), said mandrin (1') protruding from the needle (4') for tissue access;
- an adjustable sealing ring (13') with a screw-on crimped connection for gas-tight fixation of the energy conductor (16) is integrated in the through channel (12') of the catheter (10'); and
- the catheter (10') is permeable to the energy released by the energy conductor (16) so that the energy conductor can be located inside the catheter (10') during treatment.

4. The tissue access and treatment system according to claim 3, **characterized in that** the catheter (10') is longer than the needle (4') and comprises a marking (14) for positioning its distal end relative to the distal end of the needle (4').

5. The tissue access and treatment system according to claim 2 or 3, **characterized in that** the catheter (10, 10'), at least in the section where the energy-releasing zone of the energy conductor (16) is located, consists of heat-resistant material that is permeable to the energy released by the energy conductor (16).

6. The tissue access and treatment system according to any one of claims 1 through 5, **characterized in that** the energy conductor (16) comprises a diffusing body at its energy-releasing distal end.

7. The tissue access and treatment system according to any one of claims 1 through 6, **characterized in that** a stop (5, 5') that can be adjusted in longitudinal direction is mounted to the needle (4, 4') to limit puncture depth.

8. The tissue access and treatment system according to any one of claims 1 through 7, **characterized in that** a sterile pharmaceutical sealing liquid is present in the remaining narrowest gaps between the needle (4, 4') and the mandrin (1) to prevent air from entering the organ to be treated.

9. The tissue access and treatment system according to any one of claims 1 through 8, **characterized in that** the energy-transparent needle and catheter material is also permeable to sound as well as long-wave and infrared light.

10. The tissue access and treatment system according to any one of claims 1 through 9, **characterized in that** the energy conductor (16) is connected to a laser light source or an infrared radiation source or a short-wave sound source or a vapor source.

11. The tissue access and treatment system according to any one of claims 1 through 10, **characterized in that** the infusion fitting (9, 15) is connected to a gaseous or liquid media reservoir used as a sealing agent or coolant and heat transport medium or contrast or ablation or therapeutic agent.

12. The tissue access and treatment system according to any one of claims 1 through 3, **characterized in that** the coolant flows through the annular flow duct at a rate of 0.1 to 2 ml/minute.
